# EUROPÄISCHE PATENTSCHRIFT

(11) **EP 0 626 379 B1**
(45) Veröffentlichungstag und Bekanntmachung des Hinweises auf die Patenterteilung: **21.03.2001**
(21) Anmeldenummer: 94105776.2
(22) Anmeldetag: 14.04.1994
(51) Int. Cl.: C07D 277/68, C07D 263/58

(54) **Verfahren zur Herstellung von 2-Chlor-benzothiazolen oder 2-Chlor-benzoxazolen**
Process for the preparation of 2-chlorobenzothiazole or 2-chlorobenzoxazole
Procédé de préparation du 2-chlorobenzothiazole ou du 2-chlorobenzoxazole

(30) Priorität: 27.04.1993 DE 4313748; 27.04.1993 DE 4313750
(43) Veröffentlichungstag der Anmeldung: 30.11.1994
(73) Patentinhaber: BAYER AG, 51368 Leverkusen (DE)
(72) Erfinder: Käss, Volker, Dr., D-51375 Leverkusen (DE)

(56) Entgegenhaltungen:
- DE-A- 3 207 153
- DE-B- 1 210 617
- US-A- 2 469 697
- HOUBEN-WEYL 'methoden der organischen chemie - band V/3' , EUGEN MüLLER , GEORG THIEME VERLAG - STUTTGART (1962) * Seiten 871, 858 *
- CHEMICAL ABSTRACTS, vol. 101, no. 9, 27. August 1984, Columbus, Ohio, US; abstract no. 72718s, & CS-A-213 291 (J. HROMAS) 1. Februar 1984

## Beschreibung

Die vorliegende Erfindung betrifft ein Verfahren zur Herstellung der Titelverbindungen aus den zugehörigen 2-Mercapto-Verbindungen und Thionylchlorid in Gegenwart von Phosphorigsäure-Verbindungen sowie ein Verfahren zur Gewinnung von elementarem Schwefel aus dabei erhaltenen flüssigen Reaktionsgemischen. Das erfindungsgemäße Verfahren wird bei einer Temperatur von 30-140°C durchgeführt.

Die Titelverbindungen sind in substituierter oder unsubstituierter Form wichtige Zwischenprodukte, beipielsweise zur Herstellung von Wirkstoffen im Pflanzenschutzbereich oder für Farbstoffe. So ist 2-Chlor-benzothioazol ein Zwischenprodukt für die Herstellung von Herbiziden (EP-B1-0 005 501; EP-A1-0 014 409).

Aus DE-OS 1 670 453 ist bekannt, 2-Chlor-benzothiazol, das durch Halogen oder Nitro substituiert sein kann, aus dem zugehörigen 2-Mercapto-benzothiazol oder dessen Disulfid durch Umsetzung mit Chlor in Gegenwart von inerten organischen Lösungsmitteln bei mäßig erhöhter Temperatur von 70-150°C oder vorteilhafter bei 80-90°C herzustellen. Den Ausführungsbeispielen von DE-OS'453 entnimmt man Ausbeuten von 79,5-87 % der theoretischen Ausbeute an 2-Chlor-benzothiazol.

Ein weiteres Verfahren zur Herstellung von 2-Chlor-benzothiazol durch Chlorierung von 2-Mercapto-benzothiazol gemäß DE-OS 3 234 531 ist durch den Einsatz von Phosphortrichlorid und elementarem Chlor als Chlorierungsmittel und eines tertiären Amins als Katalysator gekennzeichnet. Nach diesem Verfahren lassen sich die Ausbeuten auf etwa 92 % der theoretischen Ausbeute steigern, jedoch ist dieses Verfahren in seiner Ausführung und in der Aufarbeitung des Reaktionsgemisches recht aufwendig. Aus Houben-Weyl, Methoden der organischen Chemie, Band V/3, S. 871, ist es schließlich bekannt, 2-Mercapto-benzothiazol als Aufschlämmung, in überschüssigem Phosphoroxychlorid oder Phosphortrichlorid mit Thionylchlorid zu 2-Chlor-benzothiazol umzusetzen.

Es wurde nun gefunden, daß man die Chlorierung der zugehörigen 2-Mercapto-Verbindungen zu den Titelverbindungen mit Thionylchlorid in üblichen und einfach zu handhabenden organischen Lösungs- und Verdünnungsmitteln durchführen kann, wenn man in Gegenwart einer kleinen Menge einer Phosphorigsäure-Verbindung arbeitet.

Es wurde ein Verfahren zur Herstellung von 2-Chlor-benzothiazolen oder 2-Chlor-benzoxazolen der Formel (I) 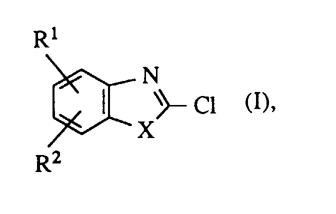 in der
- R¹ und R²: unabhängig voneinander Wasserstoff, geradkettiges oder verzweigtes C₁-C₄-Alkyl, geradkettiges oder verzweigtes C₁-C₄-Alkoxy, Nitro, Fluor, Chlor, Brom, SO₂Cl, Cyano oder SO₂-C₁-C₄-Alkyl bedeuten, und
- X: für Schwefel oder Sauerstoff steht,
durch Chlorierung von 2-Mercapto-benzothiazolen oder 2-Mercapto-benzoxazolen der Formel 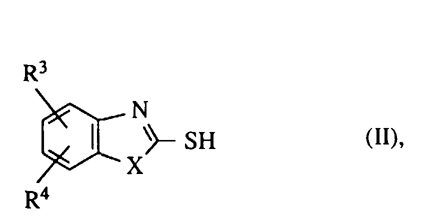 in der
- R³ und R⁴: unabhängig voneinander den Bedeutungsumfang von R¹ und R² besitzen und zusätzlich und unabhängig voneinander SO₃H bedeuten können,
mit Thionylchlorid gefunden, das dadurch gekennzeichnet ist, daß die Chlorierung unter Verwendung von 0,8-3 Mol SOCl₂ pro Mol der 2-Mercapto-Verbindung im Temperaturbereich von 30-140°C und in einem Reaktionsmedium von 50-1000 Gew.-%, bezogen auf die 2-Mercapto-Verbindung eines Lösungs- bzw. Verdünnungsmittels und unter Mitverwendung von 0,001-5 Mol-%, bezogen auf die 2-Mercapto-Verbindung, einer Phosphorigsäure-Verbindung der Formel

YP(OR⁵)₂ (III),

in der
- R⁵: Wasserstoff, geradkettiges oder verzweigtes C₁-C₁₂-Alkyl, das 1- oder 2-fach mit Halogen und/oder C₁-C₄-Alkoxy substituiert sein kann, Phenyl, das 1- oder 2-fach durch Halogen und/oder C₁-C₄-Alkyl und/oder C₁-C₄-Alkoxy substituiert sein kann, oder C₇-C₁₀-Aralkyl, dessen aromatischer Teil 1- oder 2-fach durch Halogen und/oder C₁-C₄-Alkyl und/oder C₁-C₄-Alkoxy substituiert sein kann, bedeutet, wobei im Falle zweifacher Substitution gleiche oder verschiedene Substituenten vorhanden sein können und
- Y: für Wasserstoff oder OR⁵ steht,
durchgeführt wird.

Geradkettiges oder verzweigtes C₁-C₄-Alkyl ist beispielsweise Methyl, Ethyl, Propyl, Isopropyl, Butyl, Isobutyl, bevorzugt Methyl oder Ethyl, besonders bevorzugt Methyl. Geradkettiges oder verzweigtes C₅-C₁₂-Alkyl ist eines der isomeren Pentyle, Hexyle, Octyle, Decyle oder Dodecyle.

Geradkettiges oder verzweigtes C₁-C₄-Alkoxy ist beispielsweise Methoxy, Ethoxy, Propoxy, Isopropoxy, Butoxy, Isobutoxy, bevorzugt Methoxy oder Ethoxy, besonders bevorzugt Methoxy.

C₁-C₄-Alkyl im Substituenten SO₂-C₁-C₄-alkyl hat die obengenannte Bedeutung.

C₇-C₁₀-Aralkyl ist beispielsweise Benzyl, α- und β-Phenyl-ethyl, Phenyl-propyl oder Phenyl-butyl, bevorzugt Benzyl.

Alkyl, Phenyl oder Aralkyl können 1- oder 2-fach durch Halogen, wie Fluor, Chlor oder Brom, bevorzugt Chlor, durch C₁-C₄-Alkyl oder C₁-C₄-Alkoxy substituiert sein. Für den Fall einer 2-fachen Substitution sind auch zwei verschiedene Substituenten zulässig.

In bevorzugter Form werden 2-Mercapto-Verbindungen der Formel 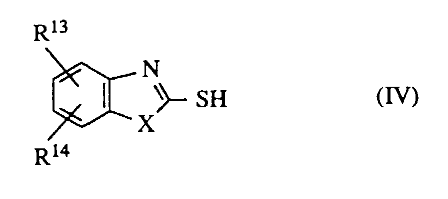 chloriert, in der
- R¹³ und R¹⁴: unabhängig voneinander Wasserstoff, Methyl, Ethyl, Methoxy, Ethoxy, Fluor oder Chlor bedeuten und
- X: für Schwefel oder Sauerstoff steht.

In besonders bevorzugter Weise werden im erfindungsgemäßen Verfahren 2-Mercapto-Verbindungen der Formel 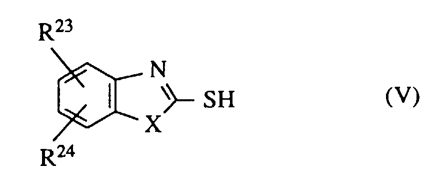 chloriert, in der
- R²³ und R²⁴: unabhängig voneinander Wasserstoff, Methyl oder Chlor bedeuten und
- X: für Schwefel oder Sauerstoff steht.

In weiterhin bevorzugter Weise werden 2-Mercapto-Verbindungen im erfindungsgemäßen Verfahren umgesetzt, in denen X für Schwefel steht.

In weiterhin bevorzugter Weise werden 2-Mercapto-Verbindungen im erfindungsgemäßen Verfahren umgesetzt, in denen der Benzolkern nur einen Substituenten trägt.

Bevorzugte Phosphorigsäure-Verbindungen für das erfindungsgemäße Verfahren sind solche, in denen Y für OR⁵ steht.

Besonders bevorzugte Phosphorigsäure-Verbindungen für das erfindungsgemäße Verfahren sind solche der Formel

P(OR¹⁵)₃ (VI),

in der
- R¹⁵: Wasserstoff, geradkettiges oder verzweigtes C₁-C₁₂-Alkyl, Phenyl, Chlorphenyl, Tolyl oder Benzyl bedeutet.

Das erfindungsgemäße Verfahren läßt sich formelmäßig am Beispiel der Umsetzung des nicht substituierten Mercapto-Benzothiazols wie folgt darstellen:

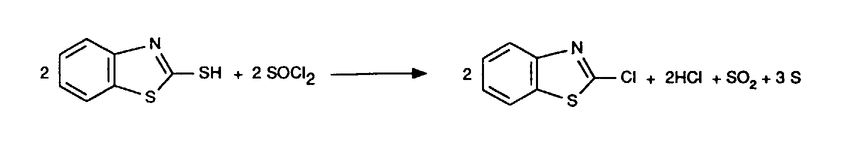

Das erfindungsgemäße Verfahren kann mit einer Thionylchloridmenge von 0,8-3 Mol, bevorzugt 0,9-1,5 Mol, besonders bevorzugt 1-1,2 Mol pro Mol der 2-Mercapto-Verbindung durchgeführt werden. Wird Thionylchlorid in einer Menge der Untergrenze von 0,8 Mol eingesetzt, birgt dies die Gefahr eines unvollständigen Umsatzes. Wird Thionylchlorid in einer Menge oberhalb der Obergrenze von 3 Mol eingesetzt, ist dies grundsätzlich möglich, bedingt jedoch das Recyclisieren einer größeren Menge nicht verbrauchten Thionylchlorids und produziert unnötig große Schwefel-Mengen als Rückstand

Die oben beschriebene Phosphorigsäure-Verbindung wird erfindungsgemäß in einer Menge von 0,001-5 Mol-%, bevorzugt 0,001-1 Mol-%, besonders bevorzugt 0,01-0,8 Mol-%, bezogen auf die 2-Mercapto-Verbindung, eingesetzt.

Lösungs- bzw- Verdünnungsmittel für das erfindungsgemäße Verfahren sind beispielsweise (cyclo)aliphatische und aromatische (Halogen)Kohlenwasserstoffe, wie C₅-C₁₂-Aliphaten, deren Gemische, wie Ligroin, Petrolether, Benzin, Kerosin; Benzol, Toluol, Xylol; Methylenchlorid, Chloroform, Tetrachlorkohlenstoff, chlorierte C₂-C₈-Aliphaten, sowie bromierte und fluorierte Analoga, Chlorbenzol, Dichlorbenzol, Trichlorbenzol, Chlortoluol, Dichlortoluol, Chlorxylol und bromierte und fluorierte Analoga sowie deren Gemische, wobei chlorierte Kohlenwasserstoffe unter den halogenierten bevorzugt sind; Ester, Ether, Nitro-aromaten, wie Ethylacetat, Butylacetat und analoge Propionate und Butyrate, Diethylether, Methylbutylether, Methyl-tert.-butyl-ether (MTBE), Tetrahydrofuran, Dioxan, Nitrobenzol, Nitrotoluol und analoge, dem Fachmann bekannte; Ketone, wie Aceton, Methylethylketon und andere. Schließlich kann auch das Reaktionsprodukt als systemeigenes Lösungs- bzw. Verdünnungsmittel eingesetzt werden, sofern es bei der Reaktionstemperatur flüssig ist.

Lösungs- bzw. Verdünnungsmittel oder ein Gemisch mehrerer von ihnen wird in einer Menge von 50-1000 Gew.-%, bevorzugt 70-700 Gew.-%, besonders bevorzugt 80-500 Gew.-%, bezogen auf die 2-Mercapto-Verbindung, eingesetzt.

Das erfindungsgemäße Verfahren wird im Temperaturbereich von 30-140°C durchgeführt. In bevorzugter Weise wird zunächst, etwa bis zur Zugabe einer äquimolaren Menge an SOCl₂, im Bereich von 30-70°C, besonders bevorzugt im Bereich von 50-65°C gearbeitet; danach wird die Temperatur in den Bereich von 60-140°C, bevorzugt 70-120°C, angehoben. Der Druck ist grundsätzlich unkritisch; bei niedrig siedenden Lösungs- bzw. Verdünnungsmitteln kann ein erhöhter Druck zur Aufrechterhaltung einer flüssigen Phase herangezogen werden.

Die Aufarbeitung des Reaktionsansatzes erfolgt durch fraktionierte Destillation, zunächst unter Normaldruck, dann unter Vakuum.

Die 2-Mercapto-Verbindungen als Ausgangsmaterial weisen in vielen Lösungsmitteln eine sehr schlechte Löslichkeit auf. Verfahren des Standes der Technik versuchten, diesen Zustand durch die Wahl besonderer Reaktionsmedien bald nach Beginn der Reaktion zu überwinden. Demgegenüber liegt im erfindungsgemäßen Verfahren lange und bis fast zur Beendigung der Chlorierung ein heterogenes Reaktionsgemisch vor. Dennoch ist in überraschender Weise eine ausreichende Reaktivität gewährleistet, die es erlaubt, inerte und leicht handhabbare Lösungs- bzw. Verdünnungsmittel einzusetzen.

Das erfindungsgemäße Verfahren erlaubt die Herstellung der Titelverbindungen in hohen Ausbeuten und hoher Reinheit. So wird beispielsweise das nicht substituierte 2-Chlor-benzothiazol bei der destillativen Aufarbeitung in einer Reinheit von mehr als 99 % in der Hauptfraktion und unter Berücksichtigung aller recyclisierbaren weiteren Fraktionen in einer Gesamtausbeute von bis zu 95 % erhalten.

Der bei der Reaktion in großen Mengen anfallende nullwertige, elementare Schwefel entsteht durch Komproportionierung. Häufig fällt entstehender elementarer Schwefel bei derartigen Reaktionen in Form von feinverteiltem, gelegentlich auch kolloidalem Schwefel an, der sich schwierig filtrieren läßt und somit die Aufarbeitung in den meisten Fällen empfindlich stört Alle Aufarbeitungsrückstände mit elementarem Schwefel sind darüber hinaus mit organischen Verunreinigungen vergesellschaftet, die es im allgemeinen nur erlauben, eine Entsorgung durch Verbrennung vorzunehmen. In einem solchen Fall fällt der Schwefel als SO₂ an und muß nach heutigem ökologischem Verständnis mit Hilfe einer Rauchgasentschwefelung entsorgt werden, wobei Gips in Mengen und Qualitäten anfällt, die eine weitere Verwendung häufig in Frage stellen.

Es wurde nun gefunden, daß man den elementaren Schwefel in kristalliner Form gewinnen kann, wenn man bei der Aufarbeitung des Reaktionsgemisches durch Destillation diese Destillation so leitet, daß ein flüssiger Destillationsrückstand erhalten bzw. aufrechterhalten wird, dessen flüssiger Zustand mit Ausnahme des kristallisierenden Schwefels auch bei Abkühlung aufrechterhalten wird.

Die Erfindung betrifft daher ebenfalls ein Verfahren zur Gewinnung von elementarem Schwefel aus flüssigen Reaktionsgemischen, die durch Umsetzung von Mercapto-Verbindungen mit Thionylhalogeniden in einem organischen Lösungsmittel entstehen, das dadurch gekennzeichnet ist, daß nach destillativer Abtrennung von mindestens einem Teil der destillierbaren Anteile unter Zurücklassung eines ebenfalls flüssigen Destillationsrückstandes dieser Destillationsrückstand unter Aufrechterhaltung seines mit Ausnahme des auskristallierenden Schwefels flüssigen Zustandes um 20-120°C, bevorzugt um 30-100°C abgekühlt wird und der auskristallisierte Schwefel mechanisch abgetrennt wird.

Dieses erfindungsgemäße Verfahren ist grundsätzlich auf alle Mercapto-Verbindungen anwendbar, aus denen durch Umsetzung mit einem Thionylhalogenid, wie Thionylchlorid oder Thionylbromid, bevorzugt Thionylchlorid, die zugehörige Halogen-Verbindung hergestellt wird. Wichtig ist diese Umsetzung insbesondere in allen Fällen, in denen von der Synthese der Ausgangsprodukte her eine MercaptoGruppe vorliegt, die sodann in eine Halogen-Gruppe umgewandelt werden soll. Dies ist insbesondere bei Heterocyclen und hier besonders bei Benzothiazol- und Benzoxazol-Verbindungen der Fall.

Für die Gewinnung elementaren Schwefels werden daher als Mercapto-Verbindungen bevorzugt 2-Mercapto-benzothiazole und 2-Mercapto-benzoxazole der Formel 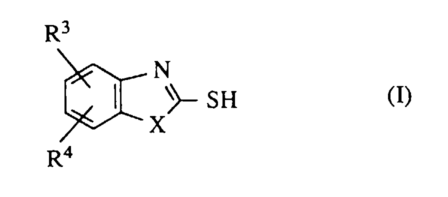 eingesetzt, in der
- R³ und R⁴: unabhängig voneinander Wasserstoff, geradkettiges oder verzweigtes C₁-C₄-Alkyl, geradkettiges oder verzweigtes C₁-C₄-Alkoxy, Nitro, Fluor, Chlor, Brom, SO₂Cl, SO₃H, Cyano oder SO₂-C₁-C₄-Alkyl bedeuten und
- X: für Schwefel oder Sauerstoff steht.

Weitere bevorzugte Ausführungsformen zur Gewinnung elementaren Schwefels entsprechen hinsichtich der zum Einsatz kommenden Mercaptoverbindungen denen, die zur Herstellung der bevorzugten Titelverbindungen verwendet werden.

Als Lösungs- bzw. Verdünnungsmittel für die erfindungsgemäß zu behandelnden Reaktionsgemische kommen neben den schon oben genannten für den Zweck der Schwefelgewinnung auch die nachstehenden in Frage: Acetonitril; niedere Dialkylcarbonate, wie Dimethylcarbonat; peralkylierte Säureamide, wie Dimethylformamid (DMF), Dimethylacetamid (DMA), N-Methyl-pyrrolidon (NMP), N-Methyl-caprolactam (NMC); N-substituierte Morpholine; Phosphorhalogenide, wie Phosphoroxichlorid, Phosphortrichlorid; weitere, dem Fachmann aus der einschlägigen Literatur bekannte Lösungsmittel, sofern sie bei der Reaktionstemperatur der Chlorierung und der anschließenden erfindungsgemäßen Behandlung flüssig und inert sind.

In bevorzugter Form werden als Lösungs- bzw. Verdünnungsmittel die obengenannten aliphatischen und aromatischen (Halogen)Kohlenwasserstoffe, Ester, Ether, Nitroaromaten, besonders bevorzugt aliphatische und aromatische (Halogen)Kohlenwasserstoffe eingesetzt.

Die erfindungsgemäß zur Gewinnung von elementarem Schwefel zu behandelnden Reaktionsgemische können ferner Reaktionsbeschleuniger, wie tertiäre Amine, Phosphite oder andere enthalten. Bevorzugte Beschleuniger dieser Art sind die oben genannten Phosphorigsäure-Verbindungen der Formel (III) und ihre weiteren bevorzugten Ausführungsformen.

Für die zwecks Schwefelgewinnung hergestellten Reaktionsgemische werden solche Phosphite im allgemeinen in einer Menge von 0,01-5 Mol-%, bevorzugt 0,02-1 Mol-%, besonders bevorzugt 0,04-0,8 Mol-%, bezogen auf die Mercapto-Verbindung, eingesetzt. Die Halogenierung der Mercapto-Verbindung mit Thionylhalogenid in Gegenwart eines solchen Phosphits wird beispielsweise im Temperaturbereich von 30-140°C durchgeführt.

Das erfindungsgemäß zur Gewinnung von elementarem Schwefel aufzuarbeitende Reaktionsgemisch wird destillativ behandelt. Hierbei wird in einem Vorlauf zunächst überschüssiges Thionylhalogenid und Lösungsmittel abgetrennt, woraufhin bei verbessertem Vakuum die destillative Gewinnung des halogenierten, aus der Mercapto-Verbindung hervorgegangenen Produkts erfolgt. Gemäß Stand der Technik wird danach ein Destillationssumpf erhalten, der beim Erkalten zu einer zähen Masse wird, die teilweise erstarrt. Zur leichteren Handhabung dieses Destillationssumpfes, beispielsweise zur Einspeisung in eine Verbrennungsanlage mit Rauchgasentschwefelung, hält man diesen Destillationssumpf bevorzugt und mit dem dazu erforderlichen Aufwand bei erhöhter Temperatur im flüssigen Zustand, so daß er in einer Verbrennungsanlage durch Pumpen bewegt werden kann.

Erfindungsgemäß wird nun das aufzuarbeitende Reaktionsgemisch destillativ von mindestens einem Teil der destillierbaren Anteile befreit und anschließend um 20-120°C, bevorzugt um 30-100°C abgekühlt, wobei der Schwefel in kristalliner Form ausfallt. Hierbei muß mit Ausnahme des auskristallisierenden Schwefels der übrige Destillationsrückstand flüssig bleiben. Dies kann beispielsweise in zwei Varianten erreicht werden:
a) Dem Reaktionsgemisch wird zunächst gegebenenfalls nicht umgesetztes Thionylhalogenid und das Lösungsmittel oder ein Gemisch mehrerer von ihnen als Vorlauf und sodann das aus der Mercapto-Verbindung hervorgegangene halogenierte Produkt als Hauptlauf entnommen. Sodann wird der noch heiße Destillationssumpf mit einem Lösungsmittel der obengenannten Art, beispielsweise mit dem Vorlauf, verdünnt. In bevorzugter Weise wird hierzu der Vorlauf zu 50-100 % seiner Gesamtmenge, bevorzugt zu 80-100 % seiner Gesamtmenge, eingesetzt. Selbstverständlich ist es möglich, über den Vorlauf hinaus zusätzliches Lösungsmittel dem heißen Destillationssumpf zuzusetzen. Im allgemeinen wird davon nur Gebrauch gemacht, um Verluste an Lösungsmittel auszugleichen. Nach Abkühlung auf den obengenannten Temperaturbereich läßt sich der auskristallisierte Schwefel mechanisch abtrennen, beispielsweise durch Kristallisieren, Dekantieren, Zentrifugieren oder durch ähnliche Operationen. Der mechanisch abgetrennte kristalline Schwefel wird sodann mit Lösungsmittel oder mit Wasser gewaschen und kann nach einer Trocknung einem technischen Einsatzzweck, beispielsweise der Schwefelsäure-Gewinnung, zugeführt werden. Auf diese Weise läßt sich ein Schwefel mit einer Reinheit von oberhalb 98 % und in einer Menge von mehr als 80 % des gesamten im Destillationssumpf vorhandenen Schwefels gewinnen. Die aus der mechanischen Abtrennung des kristallinen Schwefels hervorgehende Mutterlauge besteht im wesentlichen aus dem eingesetzten Lösungsmittel, beispielsweise dem eingesetzten Destillationsvorlauf, und enthält weiterhin etwa zugesetzte Reaktionsbeschleuniger, wie ein obengenanntes Phosphit, sowie restliches Reaktionsprodukt und eventuell nicht umgesetzte Mercapto-Verbindung und kann in dieser Form dem nächsten Ansatz zur Halogenierung weiterer Mercapto-Verbindungen als Lösungs- bzw. Verdünnungsmittel zugesetzt werden. Entsprechend den Löslichkeitsverhältnissen liegt weiterhin restlicher, nicht auskristallisierter Schwefel vor.
b) Ein Reaktionsansatz aus der Halogenierung einer Mercapto-Verbindung wird destillativ lediglich von einem Vorlauf, bestehend aus nicht umgesetztem Thionylhalogenid und dem Lösungsmittel, sowie gegebenenfalls von einem Zwischenlauf, befreit. Der Rest des so behandelten Reaktionsgemisches, der noch das aus der Mercapto-Verbindung hervorgegangene halogenierte Reaktionsprodukt enthält, wird sodann auf eine Temperatur im oben angegebenen Bereich abgekühlt, woraufhin auch bei dieser Variante Schwefel in kristalliner Form ausfällt. Nach der mechanischen Abtrennung des Schwefels in der oben angegebenen Form wird der als Mutterlauge anfallende Rest des Reaktionsgemisches destillativ weiter behandelt, wobei das halogenierte Reaktionsprodukt erhalten wird (im allgemeinen in einer Vakuumdestillation) und ein weitgehend vom Schwefel befreiter Destillationssumpf in nunmehr nur geringer Menge erhalten wird. Dieser Destillationssumpf in geringer Menge bereitet, was sowohl seine geringere Menge als auch seinen stark verminderten Gehalt an Schwefel und die Notwendigkeit einer Rauchgasentschwefelung angeht, nunmehr wesentlich verringerten Aufwand.

Das erfindungsgemäße Verfahren ist ein bedeutender Beitrag zur Entlastung der Umwelt von chemischen Rückständen, wobei durch nur unwesentlichen Destillationsaufwand ein bedeutend höherer Aufwand an Energieeinsatz und Manipulation zur anderweitigen Rückstandsentsorgung eingespart wird.

Die Abkühlung des durch Destillation eingeengten Reaktionsgemisches kann beispielsweise auf einen Temperaturbereich von 40-70°C, bevorzugt 45-55°C vorgenommen werden. Dieser Temperaturbereich hat sich beim Einsatz von Halogenaromaten als Lösungsmittel als vorteilhaft herausgestellt Ganz allgemein ist der günstige Bereich vom Siedepunkt des Lösungsmittels und vom Kristallisationsbeginn des Schwefels abhängig und wird durch routinemäßige Vorversuche ermittelt.

### Beispiel 1

Ein 4 1-Vierhalskolben war mit Rührer, Thermometer, Kühler und Tropftrichter mit langem Tauchrohr ausgerüstet. In eine vorgelegte Suspension von 1017,9 g 2-Mercapto-benzothiazol (6 Mol) und 3,5 g Triphenylphosphit in 1200 ml = 1320 g Chlorbenzol leitete man über das Tauchrohr innerhalb von etwa 6 Stunden bei 60-65°C 489 ml = 802,8 g (6,7 Mol) SOCl₂ ein. Die Dosiergeschwindigkeit folgte hierbei der starken Gasentwicklung. Die anfänglich sehr dicke Suspension wurde erst im Verlaufe der SOCl₂-Zugabe dünner und besser rührbar; am Ende lag eine Lösung vor. Nach Zugabe des SOCl₂ wurde 3 Stunden bei 90°C nachgerührt. Danach wurde eine bei Normaldruck beginnende und bei Unterdruck fortgesetzte fraktionierte Destillation zur Gewinnung von Chlorbenzol und dem bei etwa 140°C/35 mbar siedenden 2-Chlor-benzothiazol (2-CBT) durchgeführt. Folgende Bedingungen wurden eingestellt; folgende Ergebnisse wurden erhalten:

| | **Sumpf** | **Kopf** | **Heizbad** | **Druck** |
|---|---|---|---|---|
| **Vorlauf** | bis 120°C | bis 70°C | bis 144°C | 1000 bis 25 mbar |
| **Zwischenlauf** | bis 134°C | bis 122°C | bis 162°C | 25 mbar |
| **Hauptlauf** | bis 208°C | 125-70°C | bis 220°C | 20 mbar, |

| | **Masse (g)** | **Gehalt an 2-CBT** | | **Theoret. Ausbeute (%)** |
|---|---|---|---|---|
| | | **(%)** | **(g 100 %ig)** | |
| **Vorlauf** | 1339,0 | 0,1 | 1,3 | 0,1 |
| **Zwischenlauf** | 14,2 | 53,7 | 7,6 | 0,7 |
| **Hauptlauf** | 907,8 | 99,3 | 901,6 | 88,6 |
| **Sumpf** | 384,9 | | | |
| **Kühlfalle** | 170,4 | | | |
| **Summe** | 2816,3 | | 910,5 | 89,4 |

Das Reaktionsabgas wurde in verdünnte Natronlauge geleitet Der Rückstand enthielt neben den Elementen C, H, N, Cl, O und P etwa 76 Gew.-% Schwefel. Der Rückstand wurde mit einer Temperatur von etwa 100°C aus dem Kolben abgelassen; der Kolben wurde mit 100 ml o-Dichlorbenzol ausgespült und so für den nächsten Ansatz gereinigt. Die Wiederholung des Ansatzes mit o-Dichlorbenzol als Lösungs-/Verdünnungsmittel führte zu vergleichbaren Ergebnissen.

### Beispiel 2

Der mit o-Dichlorbenzol verdünnte Rückstand aus Beispiel 1 wurde in einer mit Rauchgasentschwefelung ausgerüsteten Rückstandsentsorgungsanlage verbrannt.

### Beispiel 3

Beispiel 1 wurde wiederholt, wobei jedoch der Destillationsrückstand im Destillationskolben belassen wurde. Nach Abkühlung dieses Destillationssumpfes auf etwa 120-130°C wurde der gesamte vorher entnommene Vorlauf zugesetzt und das so vereinigte Gemisch auf 45-50°C abgekühlt. Hierbei kristallisierte Schwefel aus, der abfiltriert wurde und dreimal mit 50 ml Chlorbenzol nachgewaschen wurde. Nach dem Trocknen erhielt man 260 g Schwefel mit einer Reinheit von 98,7 %. Die hierbei anfallende Mutterlauge wurde als Lösungsmittel, das gleichzeitig den Reaktionsbeschleuniger Phosphit enthält, dem nächsten Reaktionsansatz zugesetzt, wobei vergleichbare Resultate erhalten wurden.

### Beispiel 4

Das Beispiel 1 wurde wiederholt. Dem Reaktionsgemisch wurde jedoch nur das Lösungsmittel als Vorlauf entnommen. Hierbei wurde nur bis zu einer Sumpftemperatur von 120°C und einem Vakuum von bis zu 200 mbar gearbeitet Der vom Vorlauf befreite Reaktionsansatz wurde sodann, wie im Beispiel 3 angegeben, auf 45-50°C abgekühlt und in der im Beispiel 3 angegebenen Weise vom auskristallisierten Schwefel befreit. Die hierbei erhaltene Mutterlauge, die mit dem zum Waschen des abfiltrierten Schwefels benutzten Chlorbenzol vereinigt wurde, wurde erneut der Destillation unterworfen, worin nach Entnahme eines Zwischenlaufs der im Beispiel 1 angegebene Hauptlauf erhalten wurde. Der danach verbleibende Destillationssumpf betrug 96,5 g und damit nur etwa 1/4 des in Beispiel 1 erhaltenen Destillationssumpfs.

## Patentansprüche

1. Verfahren zur Herstellung von 2-Chlor-benzothiazolen oder 2-Chlor-benzoxazolen der Formel 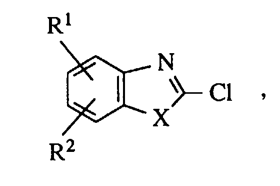 in der
R¹ und R² unabhängig voneinander Wasserstoff, geradkettiges oder verzweigtes C₁-C₄-Alkyl, geradkettiges oder verzweigtes C₁-C₄-Alkoxy, Nitro, Fluor, Chlor, Brom, SO₂Cl, Cyano oder SO₂-C₁-C₄-Alkyl bedeuten, und
X für Schwefel oder Sauerstoff steht,
durch Chlorierung von 2-Mercapto-benzothiazolen oder 2-Mercapto-benzoxazolen der Formel 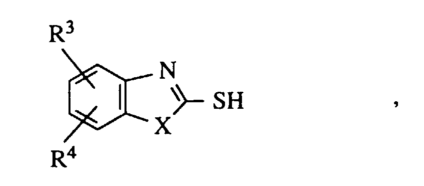 in der
R³ und R⁴ unabhängig voneinander den Bedeutungsumfang von R¹ und R² besitzen und zusätzlich und unabhängig voneinander SO₃H bedeuten können,
mit Thionylchlorid, dadurch gekennzeichnet, daß die Chlorierung unter Verwendung von 0,8-3 Mol SOCl₂ pro Mol der 2-Mercapto-Verbindung im Temperaturbereich von 30-140°C und in einem Reaktionsmedium von 50-1000 Gew.-%, bezogen auf die 2-Mercapto-Verbindung, eines Lösungs- bzw. Verdünnungsmittels und unter Mitverwendung von 0,001-5 Mol-%, bezogen auf die 2-Mercapto-Verbindung, einer Phosphorigsäure-Verbindung der Formel
YP(OR⁵)₂ ,
in der
R⁵ Wasserstoff, geradkettiges oder verzweigtes C₁-C₁₂-Alkyl, das 1-oder 2-fach mit Halogen und/oder C₁-C₄-Alkoxy substituiert sein kann, Phenyl, das 1- oder 2-fach durch Halogen und/oder C₁-C₄-Alkyl und/oder C₁-C₄-Alkoxy substituiert sein kann, oder C₇-C₁₀-Aralkyl, dessen aromatischer Teil 1- oder 2-fach durch Halogen und/oder C₁-C₄-Alkyl und/oder C₁-C₄-Alkoxy substituiert sein kann, bedeutet, wobei im Falle zweifacher Substitution gleiche oder verschiedene Substituenten vorhanden sein können und
Y für Wasserstoff oder OR⁵ steht,
durchgeführt wird.

2. Verfahren nach Anspruch 1, dadurch gekennzeichnet, daß 2-Mercaptobenzothiazole oder 2-Mercapto-benzoxazole der Formel 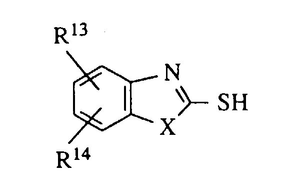 chloriert werden, in der
R¹³ und R¹⁴ unabhängig voneinander Wasserstoff, Methyl, Ethyl, Methoxy, Ethoxy, Fluor oder Chlor bedeuten und
X für Schwefel oder Sauerstoff steht,
daß bevorzugt 2-Mercapto-benzothiazole oder 2-Mercapto-benzoxazole der Formel 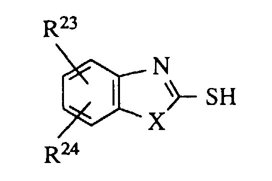 chloriert werden, in der
R²³ und R²⁴ unabhängig voneinander Wasserstoff, Methyl oder Chlor bedeuten und
X für Schwefel oder Sauerstoff steht.

3. Verfahren nach Anspruch 1, dadurch gekennzeichnet, daß die 2-Mercapto-Verbindung nur einen Substituenten trägt.

4. Verfahren nach Anspruch 1, dadurch gekennzeichnet, daß X für Schwefel steht.

5. Verfahren nach Anspruch 1, dadurch gekennzeichnet, daß 0,9-1,5 Mol, bevorzugt 1-1,2 mol SOCl₂ pro Mol 2-Mercapto-Verbindung eingesetzt werden.

6. Verfahren nach Anspruch 1, dadurch gekennzeichnet, daß eine Phosphorigsäure-Verbindung der Formel
P(OR¹⁵)₃
mitverwendet wird, worin
R¹⁵ Wasserstoff, geradkettiges oder verzweigtes C₁-C₄-Alkyl, Phenyl, Chlorphenyl, Tolyl oder Benzyl bedeutet.

7. Verfahren nach Anspruch 1, dadurch gekennzeichnet, daß 70-700 Gew.-%, bevorzugt 80-500 Gew.-%, bezogen auf die 2-Mercapto-Verbindung, an Lösungs- bzw. Verdünnungsmittel eingesetzt werden.

8. Verfahren nach Anspruch 1, dadurch gekennzeichnet, daß das Lösungs- bzw. Verdünnungsmittel eines oder mehrere aus der Gruppe der aliphatischen, cycloaliphatischen oder aromatischen Kohlenwasserstoffe, der halogenierten, aliphatischen, cycloaliphatischen oder aromatischen Kohlenwasserstoffe, der Ester, Ether oder der Nitroaromaten ist, die im Bereich der Reaktionstemperaturen flüssig sind.

9. Verfahren nach Anspruch 1, dadurch gekennzeichnet, daß im Temperaturbereich von 50-120°C gearbeitet wird.

## Claims

1. Process for preparing 2-chloro-benzothiazoles or 2-chloro-benzoxazoles of the formula 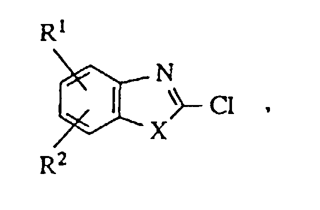 in which
R¹ and R², independently of one another, represent hydrogen, straight-chain or branched C₁-C₄-alkyl, straight-chain or branched C₁-C₄-alkoxy, nitro, fluorine, chlorine, bromine, SO₂CI, cyano or SO₂-C₁-C₄-alkyl, and
X represents sulphur or oxygen,
by chlorination of 2-mercapto-benzothiazoles or 2-mercapto-benzoxazoles of the formula 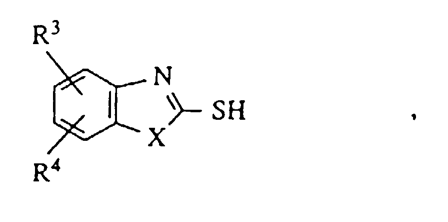 in which
R³ and R⁴, independently of one another, can have any of the meanings given for R¹ and R² and can additionally and independently of one another denote SO₃H,
with thionyl chloride, characterized in that the chlorination is carried out using 0.8-3 mol of SOCl₂ per mol of the 2-mercapto compound in the temperature range of 30-140°C and in a reaction medium of 50-1000 % by weight, based on the 2-mercapto compound, of a solvent or diluent and also using 0.001-5 mol%, based on the 2-mercapto compound, of a phosphorous acid compound of the formula
YP(OR⁵)₂,
in which
R⁵ represents hydrogen, straight-chain or branched C₁-C₁₂-alkyl which may be mono- or disubstituted by halogen and/or C₁-C₄-alkoxy, phenyl which may be mono- or disubstituted by halogen and/or C₁-C₄-alkyl and/or C₁-C₄-alkoxy, or C₇-C₁₀-aralkyl which may have its aromatic part mono- or disubstituted by halogen and/or C₁-C₄-alkyl and/or C₁-C₄-alkoxy, although in the case of disubstitution the substituents present may be identical or different, and
Y represents hydrogen or OR⁵.

2. Process according to Claim 1, characterized in that the 2-mercaptobenzothiazoles or 2-mercapto-benzoxazoles chlorinated have the formula 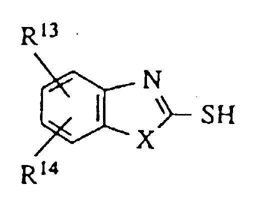 in which
R¹³ and R¹⁴, independently of one another, represent hydrogen, methyl, ethyl, methoxy, ethoxy, fluorine or chlorine and
X represents sulphur or oxygen,
preferably of the formula 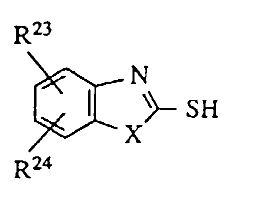 in which
R²³ and R²⁴, independently of one another, represent hydrogen, methyl or chlorine and
X represents sulphur or oxygen.

3. Process according to Claim 1, characterized in that the 2-mercapto compound bears only one substituent.

4. Process according to Claim 1, characterized in that X represents sulphur.

5. Process according to Claim 1, characterized in that 0.9-1.5 mol, preferably 1-1.2 mol, of SOCl₂ are used per mole of 2-mercapto compound.

6. Process according to Claim 1, characterized in that the phosphorous acid compound used has the formula
P(OR¹⁵)₃,
in which
R¹⁵ represents hydrogen, straight-chain or branched C₁-C₄-alkyl, phenyl, chlorophenyl, tolyl or benzyl.

7. Process according to Claim 1, characterized in that 70-700 % by weight, preferably 80-500 % by weight, based on the 2-mercapto compound, of solvent or diluent are used.

8. Process according to Claim 1, characterized in that the solvent or diluent is one or more from the group of the aliphatic, cycloaliphatic or aromatic hydrocarbons, of the halogenated, aliphatic, cycloaliphatic or aromatic hydrocarbons, of the esters, ethers or of the nitroaromatics which are liquid in the range of the reaction temperatures.

9. Process according to Claim 1, characterized in that the reaction is carried out in the temperature range of 50-120°C.

## Revendications

1. Procédé pour la préparation des 2-chloro-benzothiazoles ou 2-chloro-benzoxazoles de formule 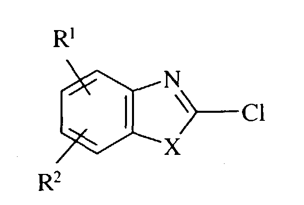 dans laquelle
R¹ et R² représentent chacun, indépendamment l'un de l'autre, l'hydrogène, un groupe alkyle à chaîne droite ou ramifiée en C₁-C₄, alcoxy à chaîne droite ou ramifiée en C₁-C₄, nitro, le fluor, le chlore, le brome, un groupe SO₂CI, cyano ou SO₂-alkyle en C₁-C₄ et
X représente le soufre ou l'oxygène,
par chloruration des 2-mercapto-benzothiazoles ou 2-mercapto-benzoxazoles de formule 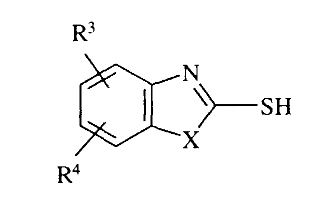 dans laquelle
R³ et R⁴ ont, indépendamment l'un de l'autre, les significations indiquées pour R¹ et R² mais peuvent en outre représenter, indépendamment l'un de l'autre, un groupe SO₃H,
à l'aide du chlorure de thionyle, caractérisé en ce que la chloruration est réalisée à l'aide de 0,8 à 3 mol de SOCI₂ par mole du dérivé en 2-mercapto dans l'intervalle de température de 30 à 140°C et dans un milieu de réaction consistant en un solvant ou diluant en proportions de 50 à 1 000 % en poids, par rapport au dérivé en 2-mercapto, avec utilisation conjointe de 0,001 à 5 mol %, par rapport au dérivé en 2-mercapto, d'un dérivé de l'acide phosphoreux de formule
YP(OR⁵)₂
dans laquelle
R⁵ représente l'hydrogène, un groupe alkyle à chaîne droite ou ramifiée en C₁-C₁₂ qui peut porter 1 ou 2 substituants halogéno et/ou alcoxy en C₁-C₄, phényle, qui peut porter 1 ou 2 substituants halogéno et/ou alkyle en C₁-C₄ et/ou alcoxy en C₁-C₄, ou aralkyle en C₇-C₁₀ dont la partie aromatique peut porter 1 ou 2 substituants halogéno et/ou alkyle en C₁-C₄ et/ou alcoxy en C₁-C₄, les substituants pouvant être identiques ou différents dans le cas d'une double substitution, et
Y représente l'hydrogène ou OR⁵.

2. Procédé selon la revendication 1, caractérisé en ce que l'on chlore des 2-mercapto-benzothiazoles ou 2-mercapto-benzoxazoles de formule 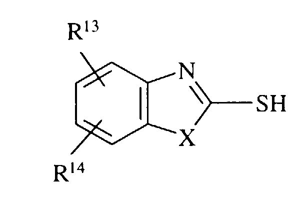 dans laquelle
R¹³ et R¹⁴ représentent chacun, indépendamment l'un de l'autre, l'hydrogène, un groupe méthyle, éthyle, méthoxy, éthoxy, le fluor ou le chlore et
X représente le soufre ou l'oxygène, de préférence des 2-mercaptobenzothiazoles ou 2-mercapto-benzoxazoles de formule 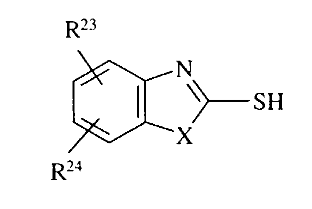
dans laquelle
R²³ et R²⁴ représentent chacun, indépendamment l'un de l'autre, l'hydrogène, un groupe méthyle ou le chlore et
X représente le soufre ou l'oxygène.

3. Procédé selon la revendication 1, caractérisé en ce que le dérivé en 2-mercapto ne porte qu'un substituant.

4. Procédé selon la revendication 1, caractérisé en ce que X représente le soufre.

5. Procédé selon la revendication 1, caractérisé en ce que l'on met en oeuvre de 0,9 à 1,5 mol, de préférence de 1 à 1,2 mol de SOCI₂ par mole du dérivé en 2-mercapto.

6. Procédé selon la revendication 1, caractérisé en ce que l'on utilise conjointement un dérivé de l'acide phosphoreux de formule
P(OR¹⁵)₃
dans laquelle
R¹⁵ représente l'hydrogène, un groupe alkyle à chaîne droite ou ramifiée en C₁-C₄, phényle, chlorophényle, tolyle ou benzyle.

7. Procédé selon la revendication 1, caractérisé en ce que l'on utilise le solvant ou diluant en proportions de 70 à 700 % en poids, de préférence de 80 à 500 % en poids, par rapport au dérivé en 2-mercapto.

8. Procédé selon la revendication 1, caractérisé en ce que le solvant ou diluant, consistant en un ou plusieurs composés du groupe des hydrocarbures aliphatiques, cycloaliphatiques ou aromatiques, des hydrocarbures aliphatiques, cycloaliphatiques ou aromatiques halogénés, des esters, des éthers ou des dérivés aromatiques nitrés, est liquide dans l'intervalle des températures de réaction.

9. Procédé selon la revendication 1, caractérisé en ce que l'on opère dans l'intervalle de température de 50 à 120°C.
